# EUROPEAN PATENT APPLICATION

(11) **EP 4 843 976 A1**
(43) Date of publication of application: **25.11.2026**
(21) Application number: 25177344.6
(22) Date of filing: 19.05.2025
(51) Int. Cl.: C07C 45/82, C07C 47/02, F25B 30/06

(54) **PROCESS FOR WORKING UP A PROCESS STREAM COMPRISING ISOBUTYRALDEHYDE AND N-BUTYRALDEHYDE**

(71) Applicant: BASF SE, 67056 Ludwigshafen am Rhein (DE)
(72) Inventor: CLAUSEN, Iven, 67056 Ludwigshafen am Rhein (DE); ARORA, Sachin Kumar, 67056 Ludwigshafen am Rhein (DE); KIRCHNER, Tanja, 67056 Ludwigshafen am Rhein (DE); PAPP, Reiner, 67056 Ludwigshafen am Rhein (DE)
(74) Representative: Kudla, Karsten

(57) **Abstract**

The invention relates to a process for working up a process stream (1) comprising isobutyraldehyde and n-butyraldehyde, the process comprising:
(a) feeding the process stream (1) into a distillation column (3) being operated at a bottom temperature in a range from 70 to 95 °C, a top temperature in a range from 60 to 80 °C and a pressure in a range from 1 to 1.5 bar(abs);
(b) withdrawing a top stream (5) comprising essentially isobutyraldehyde at the top of the distillation column (3);
(c) withdrawing a bottom stream (7) comprising essentially n-butyraldehyde at the bottom of the distillation column (3);
wherein at least a part (9) of the top stream (5) is cooled in a heat exchanger (11) of a heat pump (13) for producing steam and/or in a heat exchanger (35) of a district heating grid (37).

## Description

The invention relates to a process for working up a process stream comprising isobutyraldehyde and n-butyraldehyde, the process comprising:
(a) feeding the process stream into a distillation column being operated at a bottom temperature in a range from 70 to 95 °C, a top temperature in a range from 60 to 80 °C and a pressure in a range from 1 to 1.5 bar(abs);
(b) withdrawing a top stream comprising essentially isobutyraldehyde at the top of the distillation column;
(c) withdrawing a bottom stream comprising essentially n-butyraldehyde at the bottom of the distillation column.

Butyraldehyde is an important compound that is used for example in the production of additives for polymers like vulcanization accelerators or plasticizer. Further, butyraldehyde is used for producing n-butanol in industrial scale.

For producing butyraldehyde, propene and a carbon monoxide and hydrogen containing synthesis gas are fed into a hydroformylation reactor, in which the propene reacts with the carbon monoxide and hydrogen, thereby forming an n-butyraldehyde and isobutyraldehyde containing crude product stream.

For obtaining n-butyraldehyde and isobutyraldehyde, the crude product stream is worked up. In a first stage, gaseous components, particularly carbon-monoxide and hydrogen, and non-reacted propene are removed. In a following distillation process, the n-butyraldehyde and iso-butyraldehyde are separated, wherein n-butyraldehyde is obtained as bottom product and iso-butyraldehyde as top product of a distillation column.

The isobutyraldehyde withdrawn at the top of the distillation column is condensed and may be collected in a product container for isobutyraldehyde. For setting the temperature at the top of the distillation column, usually a part of the condensed isobutyraldehyde is recycled at the top into the distillation column.

For cooling usually a cooler operated with air or water as cooling medium is used.

It was an object of the present invention to provide a process for working up a process stream comprising isobutyraldehyde and n-butyraldehyde in which less energy is wasted by removing it with a cooling medium.

This object is achieved by a process for working up a process stream comprising isobutyraldehyde and n-butyraldehyde, the process comprising:
(a) feeding the process stream into a distillation column being operated at a bottom temperature in a range from 70 to 95 °C, a top temperature in a range from 60 to 80 °C and a pressure in a range from 1 to 1.5 bar(abs);
(b) withdrawing a top stream comprising essentially isobutyraldehyde at the top of the distillation column;
(c) withdrawing a bottom stream comprising essentially n-butyraldehyde at the bottom of the distillation column;
wherein at least a part of the top stream is cooled in a heat exchanger of a heat pump for producing steam and/or in a heat exchanger of a district heating grid.

By cooling at least a part of the top stream comprising essentially isobutyraldehyde, preferably the whole top stream comprising essentially isobutyraldehyde, in the heat exchanger of the heat pump for producing steam or in the heat exchanger of a district heating grid, heat of the top stream that usually is discarded by using air or cooling water for cooling the top stream, is used as energy source for producing steam or as energy source in the district heating grid. By using the heat of the top stream as energy source for producing steam or as energy source in the district heating grid, the amount of fossil based energy that usually is used as energy source for producing steam or in the district heating grid can be reduced. By this reduction, also the carbon dioxide footprint of steam generation or heat generation for the district heating grid is reduced.

The process stream comprising isobutyraldehyde and n-butyraldehyde may be obtained in a production process for butyraldehyde. For this purpose propene and a carbon monoxide and hydrogen containing synthesis gas are fed into a hydroformylation reactor. In the hydroformylation reactor, the propene reacts with carbon monoxide and hydrogen in the presence of a catalyst, thereby obtaining an n-butyraldehyde and isobutyraldehyde containing crude product stream. Besides the n-butyraldehyde and isobutyraldehyde, the crude product stream contains gaseous reactants that did not react. After removing the gaseous reactants and further low boilers and heavy boilers, the process stream comprising isobutyraldehyde and n-butyraldehyde is obtained. Since usually isobutyraldehyde and n-butyraldehyde are used in different processes, the isobutyraldehyde needs to be separated from the n-butyraldehyde. For this purpose, the process stream comprising isobutyraldehyde and n-butyraldehyde is worked up by distillation.

For distillation, the process stream comprising isobutyraldehyde and n-butyraldehyde is fed into a distillation column. The distillation column is operated at a bottom temperature in a range from 70 to 95 °C, more preferred in a range from 80 to 90 °C and particularly in a range from 81 to 87 °C, a top temperature in a range from 60 to 80 °C, more preferred in a range from 65 to 75 °C and particularly in a range from 67 to 74 °C, and a pressure in a range from 1 to 1.5 bar(abs), more preferred in a range from 1.1 to 1.3 bar(abs) and particularly in a range from 1.15 to 1.25 bar(abs).

The distillation column may be any suitable distillation column. Preferably, a distillation column comprising internals is used. Suitable internals for example are trays, structured packings or random packings. If trays are used, the trays may be any suitable type of trays, for example sieve trays, valve trays, bubble trays.

The process stream comprising isobutyraldehyde and n-butyraldehyde preferably is fed as a side feed into the distillation column. At the bottom of the distillation column, the bottom stream comprising essentially n-butyraldehyde is withdrawn and at the top of the distillation column, the top stream comprising essentially isobutyraldehyde is withdrawn. The top stream, withdrawn from the distillation column is at least partly condensed and a part of the top stream usually is recycled into the distillation column. The part not recycled into the distillation column may be collected in a storage vessel and/or may be transported to a following process which processes the isobutyraldehyde.

For operating the distillation column, a part of the bottom stream is at least partly evaporated and recycled into the distillation column and the part not being recycled into the distillation column may be collected in a storage vessel and/or may be transported to a following process that processes butyraldehyde, particularly n-butyraldehyde.

Besides the part of the bottom stream that is not recycled into the distillation column, or as an alternative, also a part of the process stream comprising isobutyraldehyde and n-butyraldehyde may be used as a reactant in the process that processes the butyraldehyde.

The process that processes the butyraldehyde may be, for example, a process for producing butanol, particularly n-butanol. For producing butanol, the n-butyraldehyde is fed into the process as a reactant and reacts with hydrogen. By reaction, the oxygen of the aldehyde group reacts with the hydrogen, forming a hydroxyl group, thereby obtaining butanol. Particularly if a part of the process stream comprising isobutyraldehyde and n-butyraldehyde is fed into the process for producing butanol, the butanol containing crude product contains n-butanol and isobutanol. The butanol containing crude product stream subsequently usually is dehydrated and separated into n-butanol and isobutanol.

If the at least part of the top stream, preferably the whole top stream, is cooled in the heat exchanger of the heat pump for producing steam, the heat pump may be an open loop heat pump or a closed loop heat pump. Preferably, the heat pump is an open loop heat pump.

In a closed loop heat pump, by cooling the at least part of the top stream in the heat exchanger of the heat pump, a working medium is evaporated by heat transfer from the product stream. After being evaporated, the working medium is compressed. By compression, the temperature of the working medium increases. Subsequently, the working medium passes a second heat exchanger, in which heat is transferred to water. After being cooled by heat transfer to heat the water, the working medium is expanded and thereby further cooled.

The water may be at least partly evaporated in the second heat exchanger. Alternatively, the water is heated and subsequently fed into a flash apparatus, in which the water is expanded and by expanding, at least a part of the water evaporates. If only a part of the water is evaporated, the liquid and the gaseous phase are separated, the gaseous phase is withdrawn as steam and the liquid phase is recycled into the second heat exchanger for being heated and optionally at least partly evaporated.

If the water is evaporated completely in the second heat exchanger, no phase separation needs to take place.

The working medium used in the closed loop of the closed loop heat pump may be any suitable working medium, for example water, glycol-water mixtures or heat transmission liquids like ethyleneoxide, cyclobutene, 1-butyne, cis-butene, trans-butene, 1,3-butadiene, 1-butene, propyne, butane, isobutene, dimethylether, cyclopropane, ammonia, propandiene, neopentane, isobutane, R441A, R1233ZDE, R1234ZEZ, R1224YDZ, R40, R436B, R510A, R435A, R429A, RE235CB2, R152A and R440A.

In an open loop heat pump, water is heated in the heat exchanger by heat transfer from the at least part of the top stream, preferably by heat transfer from the whole top stream. In the heat exchanger, the water may be at least partly evaporated. Alternatively, the heated water may be fed into a flash apparatus and at least partly evaporated by expansion. If only a part of the water is evaporated in the heat exchanger or in the flash apparatus, the gaseous phase is separated from the liquid phase and withdrawn as steam and the liquid phase may be returned into the heat exchanger to be heated and, optionally, partly evaporated, or may be withdrawn and used as heated water. However, preferably, the liquid phase is returned into the heat exchanger.

If a flash apparatus is used, the flash apparatus may be any apparatus suitable for flash evaporation and subsequent phase separation. Preferably, the flash apparatus is a flash tank, in which saturated steam forms an upper phase and the water not being evaporated a lower liquid phase. The pressure in the flash tank may be set by using a vacuum pump or, preferably, by a compressor used for compressing the steam. The water withdrawn from the flash apparatus and recycled into the heat exchanger is compressed before being fed into the heat exchanger. As some of the water is withdrawn as steam, it is necessary that also fresh water is fed into the heat exchanger. Preferably, the fresh water and the recycled water are mixed and then fed into the heat exchanger. The fresh water and the recycled water may be mixed before being compressed in the pump or, if the pressure of the fresh water already corresponds to the pressure with which the water is to be fed into the heat exchanger, downstream the pump for compressing and feeding the recycled water into the heat exchanger. The fresh water fed into the heat exchanger preferably is degassed and demineralized water.

The water being fed into the heat exchanger of the open loop pump preferably has a pressure in a range from 1 to 4 bar(abs), more preferred in a range from 1.5 to 2.5 bar(abs) and particularly in a range from 1.9 to 2.2 bar(abs). The temperature of the water being fed into the heat exchanger preferably is in a range from 30 to 70 °C, more preferred in a range from 40 to 70 °C and particularly in a range from 50 to 65 °C.

In the heat exchanger, the water preferably is heated to a temperature in a range from 45 to 95 °C, more preferred to a temperature in a range from 55 to 85 °C and particularly to a temperature in a range from 65 to 75 °C.

For evaporating, the water stream is expanded in the flash apparatus to a pressure in a range from 0.01 to 1.0 bar(abs), more preferred to a pressure in a range from 0.1 bar(abs) and particularly to a pressure in range from 0.15 to 0.4 bar(abs).

Besides using an open loop heat pump with a flash apparatus, it is also possible and preferred, that the open loop heat pump comprises a heat exchanger, in which a water stream is evaporated by heat transfer from the top stream, and a compressor, in which the evaporated water is compressed to generate process steam. If no flash evaporator is used, it is necessary that the water being heated in the heat exchanger has pressure being low enough that at least a part of the water evaporates in the heat exchanger by heat transfer from the top stream. Preferably, the water being fed into the heat exchanger has a pressure in a range from 0.05 to 0.85 bar(abs), more preferred in a range from 0.15 to 0.6 bar(abs) and particularly in a range from 0.2 to 0.4 bar(abs).

The temperature, with which the water is fed into the heat exchanger of the open loop heat pump preferably corresponds to the temperature of the water being fed into the heat exchanger of the heat pump with flash apparatus.

The steam generated in the heat pump may be used directly without further compression, if the temperature of the steam is sufficiently high to be used as an energy source in a process, for example for heating a stream or an apparatus. Processes that may use the steam as energy source may be any processes known to a skilled person that must be heated. The steam may be used, for example, for heating a reactor or for providing heat in a distillation process.

Besides directly using the steam for heating a stream or an apparatus, it is also possible to feed at least a part of the steam into a steam grid, which provides heat for several processes.

Depending on the temperature of the steam and the intended use, it may be necessary to compress the steam to obtain compressed steam. Compressing the steam particularly may be necessary if the steam is not used directly in a process but fed into a steam grid. Further, if the steam is obtained by expansion in a flash apparatus, or if the steam is obtained by evaporation in the heat exchanger of the open loop heat pump, the pressure and, hence, the temperature of the steam generally is too low to use the steam directly, so that in these cases the steam generally is compressed before being used.

The compressed steam may be low pressure steam, medium pressure steam, or high pressure steam. Further, it is possible to dived the steam produced in the heat pump into separate streams which can be compressed to different pressures. This allows, for example, to produce medium pressure steam and high pressure steam, low pressure steam and medium pressure steam, low pressure steam and high pressure steam, or even low pressure steam, medium pressure steam and high pressure steam.

In the context of the present invention, the term "low pressure steam" means steam having a pressure in a range from 0.9 to 4 bar(abs), more preferred in a range from 1 to 2 bar(abs) and particularly in a range from 1.2 to 1.5 bar(abs), and a temperature in a range from 96 to 160 °C, more preferred in a range from 99 to 140 °C, and particularly in a range from 104 to 120 °C.

The term "medium pressure steam" means steam having a pressure in a range from 4 to 8 bar(abs), more preferred in a range from 4.5 to 7 bar(abs) and particularly in a range from 5 to 6 bar(abs), and a temperature in a range from 143 to 220 °C, more preferred in a range from 147 to 210 °C, and particularly in a range from 151 to 200 °C.

The term "high pressure steam" means steam having a pressure in a range from 8 to 40 bar(abs), more preferred in a range from 10 to 30 bar(abs) and particularly in a range from 16 to 20 bar(abs), and a temperature in a range from 170 to 280 °C, more preferred in a range from 180 to 260 °C, and particularly in a range from 201 to 240 °C.

Particularly preferably, the compressed steam has a pressure in a range from 3 to 8 bar(abs), more preferred a pressure in a range from 4.5 to 7.5 bar(abs), and particularly in a range from 5 to 7 bar(abs) and a temperature in a range from 130 to 230 °C, more preferred in a range from 140 to 220 °C, and particularly in a range from 150 to 200 °C.

If the steam needs to be compressed, compression may be carried out in any compressor known to a skilled person that is suitable for compressing steam. The compression may be carried out in only one compressor or in a cascade of at least two compression stages. More than one compression stage can be implemented within one compressor comprising more than one compression stage or in a cascade of more than one compressor comprising one or more than one compression stages each. Suitable compressors for example are integrally geared turbo compressors, radial compressors, axial compressors, screw compressors, and piston compressors.

For increasing the energy efficiency and setting the properties of the steam, particularly the temperature, it is possible to inject water before or after eat least one compressor or compression stage. Further, by injecting water, the steam is cooled and particularly between two compression stages, cooling the steam has the advantage that damages due to the high temperature of overheated steam are prevented. The water is preferably injected after each compressor or compression stage. Depending on the pressure of the steam into which the water is injected, the water may need to be compressed to a pressure above the pressure of the steam, before being injected. Besides setting the properties of the steam, it is a further advantage that by injection of water the mass flow of steam increases.

The steam produced in the heat pump and the optional subsequent compression can be used in any process, which uses steam having the pressure of the steam generated in the heat pump or by compression. The steam may be used for example as an energy source in a process for working up a raw product stream obtained in a process for producing butanol. Further, the steam also may be used in any other process which preferably is carried out on the industrial site, the process for obtaining the process stream comprising isobutyraldehyde and n-butyraldehyde and, preferably, the process for producing butanol, are carried out. Besides using the steam directly in a process, preferably on the same industrial site, the steam may be fed into a steam grid.

Particularly if an open loop heat pump is used, it is preferred to compress the at least part of the top stream that is cooled in the heat exchanger of the open loop heat pump to a pressure in a range from 1 to 6 bar(abs), more preferred to a pressure in a range from 2 to 5 bar(abs) and particularly to a pressure in a range from 3 to 5 bar(abs) before using the top stream as energy source in the heat exchanger of the heat pump.

By compressing the at least part of the top stream, the temperature of the top stream rises, which has the advantage, that the water to be heated and optionally partially evaporated in the heat exchanger of the open loop heat pump may have a higher pressure, for example a pressure in a range from 0.5 to 1.5 bar(abs), more preferred in a range from 0.7 to 1.3 bar(abs) and particularly in a range from 0.9 to 1.2 bar(abs). The temperature the top stream after being compressed preferably is in a range from 60 to 140 °C, more preferred in a range from 80 to 130 °C and particularly in a range from 95 to 120 °C.

By heating the water in the heat exchanger of the heat pump, the top stream preferably is cooled to a temperature in a range from 40 to 130 °C, more preferred in a range from 45 to 75 °C, and particularly in a range from 50 to 70 °C.

Alternatively or additionally, the heat of the top stream may be used for a district heating grid. For this purpose, at least a part of the top stream is cooled in a heat exchanger of the district heating grid.

By being cooled in the heat exchanger, the at least part of the top stream, preferably the whole top stream, may transfer heat to a heating medium that is used in the district heating grid, for example, for providing heat for heating buildings or generating hot water.

However, due to the temperature of the top stream, heat transfer from the at least part of the top stream, preferably from the whole top stream, may not be sufficient for heating the heating medium of the district heating grid to a temperature that is sufficiently high, and, in this case, additional heating will be necessary. For this purpose, it is preferred that by being cooled in the heat exchanger, the at least part of the top stream transfers heat to a heat transfer medium that is heated in the heat exchanger of the district heating grid. Subsequently, the heating medium of the district heating grid in a first step is heated by heat transfer from the heat transfer medium and subsequently by additional heating.

The heat transfer medium is used for transferring the heat over the borders of the industrial site where the butyraldehyde is produced to avoid pipes through which butyraldehyde flows to cross the borders, since the district heating grid generally will be outside the industrial site.

If, however, the district heating grid is on the industrial site, for example for heating office buildings or other buildings on the industrial site, or the district heating grid is close to the industrial site, it is possible to transfer the heating medium on the industrial site and transfer heat from the at least part of the top stream to the heating medium in a heat exchanger that is located on the industrial site.

Since district heating grids usually are used for providing heat to residential areas or work areas outside the industrial site on which the butyraldehyde is produced, it is preferred that a heat transfer medium is heated in the heat exchanger of the district heating grid and the heat transfer medium then is used as a heat source for heating the heating medium that is distributed in the district heating grid. The heat transfer medium may be any suitable heat transfer medium. Preferably, the heat transfer medium is water.

Particularly if the heat transferred to the heating medium that is distributed in the district heating grid is not sufficient to heat the heating medium to a predefined temperature, it is necessary to provide additional heat for heating the heating medium. For this purpose, it is for example possible that the heat transfer medium is further heated and then used as heating medium in the district heating grid. For further heating, the heat transfer medium may be heated for example in a heater. Alternatively, if a heat transfer medium is used that at least partly evaporates by heat transfer from the top stream, further heating may be carried out by compressing the heat transfer medium before it transfers heat to the heating medium, wherein due to compression, the temperature of the heat transfer medium increases. In this case, the heat transfer medium corresponds to the working medium of a closed loop heat pump. After having transferred heat to the heating medium, the heat transfer medium is expanded, wherein due to expansion, the temperature decreases and the thus additionally cooled and expanded heat transfer medium then is recycled into the heat exchanger of the district heating grid in which the heat transfer medium is heated again by heat transfer from the top stream.

Alternatively and preferably, the heating medium may be heated in a heat pump by using heat of the heat transfer medium or may be heated by heat transfer from the heat transfer medium in a first heat exchanger and subsequent heating in a heater. Heating the heating medium in a heat pump or in a heater has the advantage, that water can be used as heat transfer medium, which, for safety reasons, is preferred.

If not the heat transfer medium is used as working medium of a heat pump but an additional heat pump is used for heating the heating medium, it is preferred to use a closed loop heat pump with a suitable working medium. In a first heat exchanger of the closed loop heat pump, the working medium is heated and at least partly evaporated, then the working medium is compressed. After compression, the working medium transfers heat to the heating medium, whereby the working medium is cooled. Subsequently, the working medium is expanded and returned into the first heat exchanger of the closed loop heat pump.

If, on the other hand, a heater is used for additionally heating the heating medium, any suitable heater can be used. Suitable heaters for example are electrical heaters, steam heaters.

If only a part of the top stream is cooled in the heat exchanger of the heat pump and/or in the heat exchanger of the district heating grid, the part of the top stream that is cooled and the remaining part preferably are recombined after the part of the top stream has passed the heat exchanger of the heat pump and/or the heat exchanger of the district heating grid.

To further cool the top stream, or to cool the top stream in cases where the heat pump is shut off, for example for maintenance purposes, or where only a part of the heat of the top stream is needed for providing heat in the district heating grid, for example in summer, it is preferred to place a cooler downstream the heat exchanger of the heat pump and/or the heat exchanger of the district heating grid. If only a part of the top stream passes the heat exchanger of the heat pump and/or the heat exchanger of the district heating grid, the cooler preferably is placed downstream the position at which the part of the top stream that was cooled and the remaining part of the top stream were recombined. Particularly if the top stream is stored in a storage vessel for storing isobutyraldehyde, it is preferred that the top stream is cooled to a temperature in a range from 10 to 55 °C, more preferred in a range from 15 to 50 °C, and particularly in a range from 25 to 45 °C, before being fed into the storage vessel.

Examples of the invention are shown in the figures and described in more detail in the following description.

In the figures:
- Figure 1: shows a distillation column for working up a process stream comprising isobutyraldehyde and n-butyraldehyde with a heat pump for cooling the top stream;
- Figure 2: shows a distillation column for working up a process stream comprising isobutyraldehyde and n-butyraldehyde with a heat exchanger for a district heating grid;
- Figure 3: shows a process chart of a process for working up a process stream comprising isobutyraldehyde and n-butyraldehyde and producing butanol;
- Figure 4: shows a schematic diagram of a combined process for producing butyraldehyde and butanol.

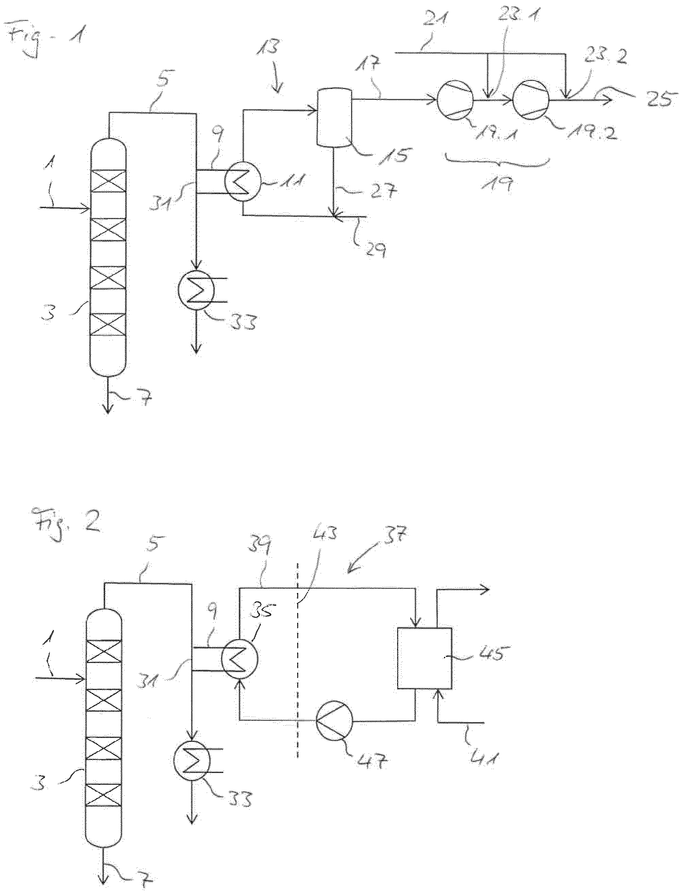

A distillation column for working up a process stream comprising isobutyraldehyde and n-butyraldehyde with a heat pump for cooling the top stream of the distillation column is shown schematically in figure 1.

For working up, a process stream 1 comprising isobutyraldehyde and n-butyraldehyde is fed into a distillation column 3. In the distillation column 3, which is operated with a bottom temperature in a range from 70 to 95 °C, a top temperature in a range from 60 to 80 °C and a pressure in a range from 1 to 1.5 bar(abs), isobutyraldehyde and n-butyraldehyde are separated. The isobutyraldehyde is withdrawn as a top stream 5 and the n-butyraldehyde as a bottom stream 7. After being cooled, the bottom stream 7 may be fed into a vessel for storing n-butyraldehyde.

The top stream 5 comprising essentially isobutyraldehyde is cooled and may be fed for example in a vessel for storing isobutyraldehyde.

For cooling, at least a part 9 of the top stream 5 flows through a heat exchanger 11 of a heat pump 13. In the embodiment shown here, the heat pump 13 is an open loop heat pump with a flash apparatus 15.

In the open loop heat pump, water is heated in the heat exchanger 11 of the heat pump by heat transfer from the at least part 9 of the top stream. The water then is fed into the flash apparatus 15, in which the water is expanded to a pressure below the boiling pressure of the heated water. By expansion, a part of the water fed into the flash apparatus 15 evaporates, thereby forming saturated steam. The saturated steam 17 is withdrawn from the flash apparatus 15 and compressed in at least one compressor 19. The at least one compressor 19 may be a compressor comprising at least two compression stages 19.1, 19.2. Alternatively, it is also possible to use more than one compressor. If more than one compressor is used, it is possible to use compressors which each have at least two compression stages, to use compressors that each have only one compression stage or at least one compressor having at least two compression stages and at least one compressor having one compression stage.

Since the steam is heated by compression, generally water 21 is fed into the steam downstream of at least one compression stage 19.1, 19.2. The water being fed into the steam evaporates, thereby cooling the steam. Further, by feeding the water, the amount of steam increases. To avoid damage of a compression stage 19.1, 19.2, each injection point 23.1, 23.2 of water is followed by an inlet path having a length that is sufficient for evaporating the whole water that was injected at the injection point 23.1, 23.2 before the steam enters the following compression stage 19.2.

After compression, compressed steam 25 is withdrawn from the last compression stage 19.2 as process steam. The compressed steam 25 may be used for example as an energy source in a process for working up a raw product stream obtained in a process for producing butanol.

The water that is not evaporated in the flash apparatus 15 is withdrawn as liquid phase 27 and preferably recycled into the heat exchanger 11 of the heat pump 13. To obtain continuous conditions in the heat pump 13, the part of the water that is withdrawn as saturated steam 17 needs to be replaced. For this purpose, fresh water 29 is fed into the liquid phase 27 recycled into the heat exchanger 15. The fresh water usually is demineralized and degassed water, for example boiler feed water.

To provide the possibility to only feed the part 9 of the top stream 5 into the heat exchanger 11 of the heat pump 13 or to bypass the heat exchanger 11 of the heat pump 13, if the heat pump may be shut off, for example for maintenance purposes, a bypass 31 is provided.

If only a part 9 of the top stream 5 passes the heat exchanger 11, the remaining part of the top stream 5 flows through the bypass 31. After having passed the heat exchanger 11, the part 9 of the top stream 5 is recombined with the remaining part of the top stream 5 that passed the bypass 31. To allow further cooling of the top stream 5, a cooler 33 may be provided. The cooler 33 particularly is used, if the top stream 5 still is too hot for further use or for being stored, after being cooled by heat transfer in the heat exchanger 11 of the heat pump 13.

Besides using an open loop heat pump with a flash apparatus 15 as shown here, it is possible to evaporate at least a part of the water in the heat exchanger 11 of the heat pump 13. If only a part of the water is evaporated in the heat exchanger 11, the partially evaporated water is fed into a phase separator for separating steam from the non-evaporated water. If the phase separator is operated at a lower pressure, additional water may evaporate on entry into the phase separator. If, on the other hand, the whole water is evaporated in the heat exchanger 11, the phase separator can be omitted.

For evaporating water at least partly in the heat exchanger 11, it is necessary that the pressure of the water fed into the heat exchanger 11, is below the boiling pressure at the temperature of the top stream 5 fed into the heat exchanger 11. To evaporate water at a higher pressure, the part 9 of the top stream 5 may be compressed before being fed into the heat exchanger 11. By compression, the temperature of the part 9 of the top stream 5 increases and, hence, the water may have a higher pressure than for being evaporated by heat transfer from the uncompressed top stream. To minimize the energy for compression, the compressor for compressing the part 9 of the top stream 5 is arranged between the position at which the part 9 branches off and the heat exchanger 11.

Figure 2 shows a distillation column for working up a process stream comprising isobutyraldehyde and n-butyraldehyde with a heat exchanger for a district heating grid.

In the embodiment shown in figure 2, the heat exchanger 11 of the heat pump 13 is replaced by a heat exchanger 35 of a district heating grid 37. In the heat exchanger 35 of the district heating grid, a heat transfer medium 39 is heated by heat transfer from the part 9 of the top stream 5.

Depending on the area in which the heat of the district heating grid is to be used, the heat transfer medium 39 may be used as heating medium of the district heating grid or the heat transfer medium 39 may be used as a heat source for heating a heating medium 41 as shown here. The heat transfer medium 39 is used as a heat source particularly if the district heating grid is outside the industrial site on which the process stream 1 comprising isobutyraldehyde and n-butyraldehyde is worked up. This is indicated in figure 2 by a dashed line 43 which symbolizes a border of an industrial site.

If the district heating grid 37 is outside the industrial site as shown here, it is important that no compounds that may be dangerous to animals or human beings or that may hazardous for the environment pass the border 43 of the industrial site. Also if the district heating grid is used for heating buildings on the industrial site, any compounds that may be dangerous should not be transported along large distances. Therefore, the heat transfer medium preferably is any liquid that is not dangerous for animals or human beings or hazardous for the environment. Particularly preferably, the heat transfer medium is water.

Depending on the distance of the district heating grid to the heat exchanger 35 of the district heating grid 37, the heat transfer medium 39 may be used as heating medium. In this case, it may be necessary to additionally heat the heat transfer medium in a heat exchanger to provide sufficient heat for heating buildings connected to the district heating grid.

Alternatively, the heat transfer medium 39 transfers heat to the heating medium 41 as shown here. However, also in this case it may be necessary to supply additional heat for heating the heating medium 41 to a temperature that is sufficiently high for heating or providing hot water in the buildings that are connected to the district heating grid.

For supplying additional heat, any suitable device 45 for supplying heat may be used. The device 45 for supplying additional heat may comprise for example a first heat exchanger in which the heating medium 41 is heated by heat transfer from the heat transfer medium 39 and subsequently, the heating medium 41 may be heated in a heater. The heater may be any suitable heater, for example an electrical heater or a heater which is operated with steam.

As an alternative, the device 45 for supplying additional heat may be a heat pump. The heat pump preferably is a closed loop heat pump. In this case, the heat transfer medium transfers heat to a working medium of the heat pump wherein at least a part of the working medium evaporates. The at least partly evaporated working medium is compressed and thereby further heated. Subsequently, the working medium is cooled by heat transfer to the heating medium 41, wherein the heating medium 41 is heated. The cooled working medium then is expanded and recycled into the heat exchanger in which the working medium is at least partly evaporated by heat transfer from the heat transfer medium.

After being used as a heat source for heating the heating medium 41, the heat transfer medium 39 is recycled into the heat exchanger 35 of the district heating grid 37. For circulating the heat transfer medium 39, a pump 47 may be used.

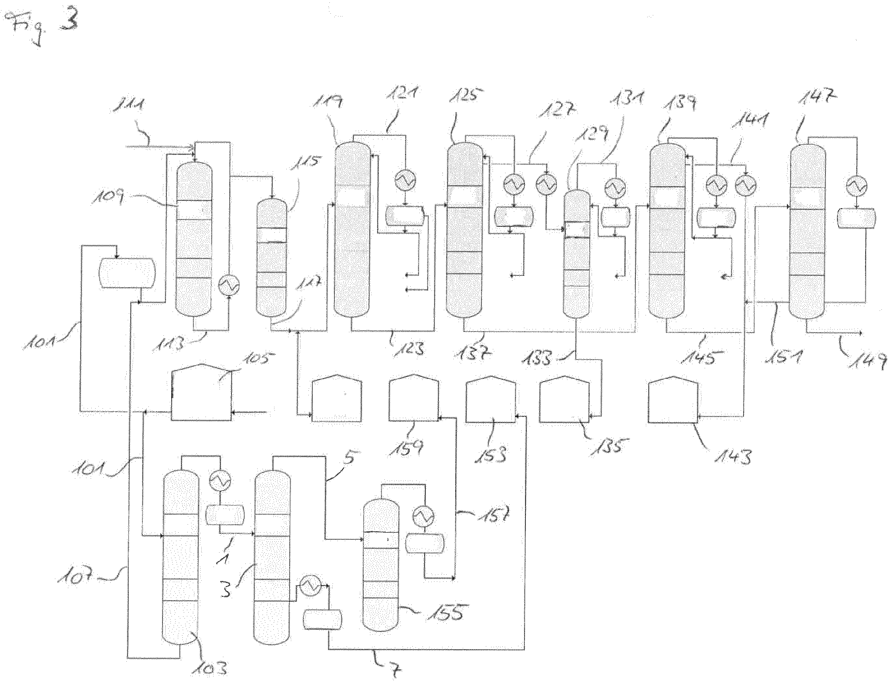

The distillation column 3 for separating the n-butyraldehyde and isobutyraldehyde generally is part of a larger process, for example a process for producing butanol. A process chart of such a process for working up a process stream comprising isobutyraldehyde and n-butyraldehyde and producing butanol is shown in figure 3.

For obtaining the process stream 1 comprising n-butyraldehyde and isobutyraldehyde, crude butyraldehyde 101 is fed into a first distillation column 103. The crude butyraldehyde 101 is obtained for example in a process for producing butyraldehyde by reacting propene, hydrogen and carbon monoxide. The crude butyraldehyde may be stored in a first storage vessel 105.

In the first distillation column 103, the crude butyraldehyde is separated in the process stream 1 comprising n-butyraldehyde and isobutyraldehyde which is withdrawn at the top of the first distillation column and bottom stream 107 comprising n-butyraldehyde and high boilers.

The bottom stream 107 and crude butyraldehyde 101 are fed into a reaction column 109, in which the butyraldehyde reacts with hydrogen 111, forming a reaction mixture 113 comprising n-butanol and isobutanol. A part of the reaction mixture 113 is recycled into the reaction column 109 and a part of the reaction mixture is fed into a second column 115 for completing the reaction to obtain crude butanol 117.

The crude butanol 117 is fed into a dewatering column 119, in which water and further low boilers are separated from the crude butanol and withdrawn as top stream 121. Dewatered crude butanol 123 is withdrawn at the bottom of the dewatering column 119 and fed into an isobutanol column 125. At the top of the isobutanol column 125 a low boilers and isobutanol comprising top stream 127 is withdrawn and fed into a column 129 for separating low boilers from the isobutanol. The low boilers 131 are withdrawn at the top of the column 129 and purified isobutanol 133 is withdrawn at the bottom of the column 129 and stored in a storage vessel 135 for isobutanol.

At the bottom of the isobutanol column 125 a stream 137 comprising n-butanol and high boilers is withdrawn and fed into an n-butanol column 139. At the top of the n-butanol column 139 purified n-butanol 141 is withdrawn and stored in a storage vessel 143 for n-butanol. At the n-butanol column 139 a bottom stream 145 comprising n-butanol and high boilers is withdrawn and fed into a final column 147 for separating high boilers 149. The high boilers 149 are withdrawn at the bottom of the final column 147 and purified n-butanol 151 is withdrawn from the top of the final column 147 and fed into the storage vessel 143 for n-butanol after being condensed and cooled.

The process stream 1 comprising isobutyraldehyde and n-butyraldehyde, withdrawn from the first distillation column 103 and fed into the distillation column 3 is separated into the top stream 5 comprising isobutyraldehyde and the bottom stream 7 comprising n-butyraldehyde. The bottom stream 7 comprising n-butyraldehyde is stored in a storage vessel 153 for n-butyraldehyde. The top stream 5 comprising isobutyraldehyde is cooled as shown in figure 1 or figure 2 and fed into a purifying column 155 for separating high boilers from the isobutyraldehyde. From the top of the purifying column 155 purified isobutyraldehyde 157 is withdrawn and stored in a storage vessel 159 for isobutyraldehyde.

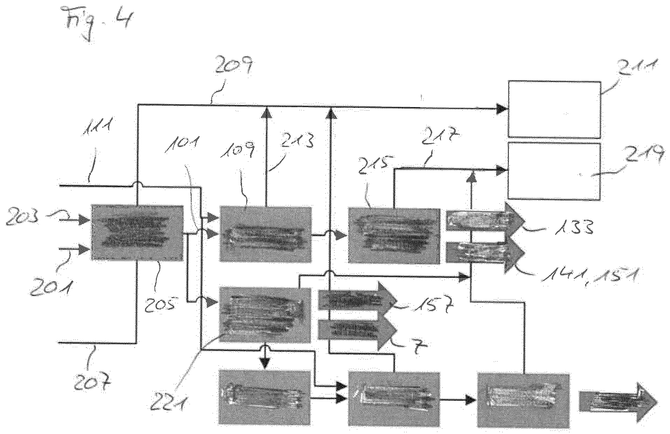

The butyraldehyde used for producing the butanol is obtained in a process for producing butyraldehyde. A schematic diagram of a combined process for producing butyraldehyde and butanol is shown in figure 4.

For producing butyraldehyde, propene 201 and a hydrogen and carbon monoxide comprising oxogas 203 are fed into an oxo-C4-synthesis 205. In the oxo-C4-sythesis, propene reacts with carbon monoxide and hydrogen, thereby forming isobutyraldehyde and n-butyraldehyde. Further, propane 207 is obtained as by-product. The propane 207 and non-reacted hydrogen and carbon monoxide are separated from crude butyraldehyde. A gas stream 209 comprising the non-reacted hydrogen and carbon monoxide may be fed for example into a process 211 for producing methanol.

A part of the crude butyraldehyde 101 and hydrogen 111 are fed into the reactor 109 for producing butanol. Non-reacted hydrogen 213 is mixed into the gas stream 209 and the crude butanol 117 is fed into the process 215 for separating n-butanol and isobutanol. The process 215 for separating n-butanol and isobutanol comprises the dewatering column 119, the isobutanol column 125, the column 129, the n-butanol column 139 and the final column 147. In the process 215for separating n-butanol and isobutanol the purified isobutanol 133 and the purified n-butanol 141, 151 are obtained.

Exhaust gas 217 obtained as low boiling components in the distillation columns 119, 125, 129, 139, 147 of the process 215 for separating isobutanol and n-butanol may be used for example as fuel in a power station 219.

The part of the crude butyraldehyde 101 that is not used for producing butanol is fed into a work-up process 221 for butyraldehyde. The work-up process 221 for butyraldehyde comprises the columns 103, 3, 155 for separating isobutyraldehyde and n-butyraldehyde. In the work-up process 221 for butyraldehyde, purified isobutyraldehyde 157 and purified n-butyraldehyde 7 are obtained.

## Claims

1. A process for working up a process stream (1) comprising isobutyraldehyde and n-butyraldehyde, the process comprising:
(a) feeding the process stream (1) into a distillation column (3) being operated at a bottom temperature in a range from 70 to 95 °C, a top temperature in a range from 60 to 80 °C and a pressure in a range from 1 to 1.5 bar(abs);
(b) withdrawing a top stream (5) comprising essentially isobutyraldehyde at the top of the distillation column (3);
(c) withdrawing a bottom stream (7) comprising essentially n-butyraldehyde at the bottom of the distillation column (3);
wherein at least a part (9) of the top stream (5) is cooled in a heat exchanger (11) of a heat pump (13) for producing steam and/or in a heat exchanger (35) of a district heating grid (37).

2. The process according to claim 1, wherein the heat pump (13) is an open loop heat pump or a closed loop heat pump.

3. The process according to claim 1 or 2, wherein the at least part (9) of the top stream (5) is compressed to a pressure in a range from 1 to 6 bar(abs).

4. The process according to claim 3, wherein after being compressed, the at least part (9) of the top stream (5) has a temperature in a range from 60 to 140 °C.

5. The process according to any of claims 1 to 4, wherein the at least part (9) of the top stream (5) is cooled to a temperature in a range from 40 to 130 °C in the heat exchanger (11) of the heat pump (13).

6. The process according to any of claims 1 to 5, wherein the steam (17) obtained in the heat pump (13) is compressed to obtain compressed steam (25).

7. The process according to claim 6, wherein the compressed steam (25) has a pressure in a range from 3 to 8 bar(abs) and a temperature in a range from 130 to 230 °C.

8. The process according to claim 6 or 7, wherein the compressed steam (25) is used as an energy source in a process for working up a raw product stream obtained in a process for producing butanol.

9. The process according to any of claims 1 to 8, wherein a heat transfer medium (39) is heated in the heat exchanger (35) of the district heating grid (37).

10. The process according to claim 9, wherein the heat transfer medium (39) is water.

11. The process according to claim 9 or 10, wherein the heat transfer medium (39) is used as a heat source for heating a heating medium (41) that is distributed in the district heating grid (37).

12. The process according to claim 11, wherein the heating medium (41) is heated in a heat pump by using heat of the heat transfer medium or is heated by heat transfer from the heat transfer medium in a first heat exchanger and subsequent heating in a heater.

13. The process according to claim 9 or 10, wherein the heat transfer medium (39) is further heated and then used as heating medium in the district heating grid.

14. The process according to any of claims 1 to 13, wherein a cooler (33) is placed downstream the heat exchanger (11) of the heat pump (13) or downstream the heat exchanger (35) of the district heating grid (37).

15. The process according to any of claims 1 to 14, wherein a part of the process stream (1) comprising isobutyraldehyde and n-butyraldehyde is used as a reactant in a process for producing butanol.
